# FASCICULE DE BREVET EUROPEEN

(11) **EP 2 780 329 B1**
(45) Date de publication et mention de la délivrance du brevet: **17.04.2019**
(21) Numéro de dépôt: 12794395.9
(22) Date de dépôt: 29.10.2012
(51) Int. Cl.: C07D 233/90, H01G 9/035, H01M 10/0568

(54) **PROCEDE DE PREPARATION DE SEL D'ANION PENTACYLIQUE**
VERFAHREN ZUR HERSTELLUNG EINES PENTAZYKLISCHEN ANIONS
PROCESS FOR THE PREPRATION OF PENTACYCLIC ANION SALT

(30) Priorité: 14.11.2011 FR 1160301
(43) Date de publication de la demande: 24.09.2014
(73) Titulaire: ARKEMA FRANCE, 92705 Colombes Cedex (FR)
(72) Inventeur: SCHMIDT, Grégory, F-69700 Saint Andéol le Château (FR); FLASQUE, Miguel, F-80090 Amiens (FR)
(74) Mandataire: Chahine, Audrey Claire
(86) Numéro de dépôt international: PCT/FR2012/052489
(87) Numéro de publication internationale: WO 2013/072591

(56) Documents cités:
- WO-A1-2010/023413
- BUKOWSKA M ET AL: "Synthesis of 4,5-dicyanoimidazoles", POLISH JOURNAL OF CHEMISTRY, POLSKIE TOWARZYSTWO CHEMICZNE, PL , vol. 78, no. 3 1 janvier 2004 (2004-01-01), pages 417-422, XP008104420, ISSN: 0137-5083 Extrait de l'Internet: URL:http://ichf.edu.pl/pjch/pj-2004/pj-200 4-03a.pdf
- NIEDZICKI L ET AL: "New type of imidazole based salts designed specifically for lithium ion batteries", ELECTROCHIMICA ACTA, ELSEVIER SCIENCE PUBLISHERS, BARKING, GB, vol. 55, no. 4, 25 janvier 2010 (2010-01-25), pages 1450-1454, XP026867410, ISSN: 0013-4686 [extrait le 2009-05-13]

## Description

### DOMAINE DE L'INVENTION

La présente invention concerne un procédé de préparation de sel d'anion pentacyclique, et notamment de 1-trifluorométhyl-4,5-dicarbonitrile-imidazolate de lithium, ainsi qu'un procédé de préparation de compositions électrolytes contenant un tel sel.

### ARRIERE-PLAN TECHNIQUE

Une batterie lithium-ion comprend au moins une électrode négative, une électrode positive, un séparateur et un électrolyte. L'électrolyte est constitué d'un sel de lithium dissous dans un solvant qui est généralement un mélange de carbonates organiques, afin d'avoir un bon compromis entre la viscosité et la constante diélectrique.

Parmi les sels les plus utilisés figure l'hexafluorophosphate de lithium (LiPF6), qui possède beaucoup des nombreuses qualités requises mais présente le désavantage de se dégrader sous forme de gaz d'acide fluorhydrique. Cela pose des problèmes de sécurité, notamment dans le contexte de l'utilisation prochaine des batteries lithium-ion pour les véhicules particuliers.

D'autres sels ont donc été développés pour fournir des électrolytes de batteries Li-ion, et notamment le LiTDI (1-trifluorométhyl-4,5-dicarbonitrile-imidazolate de lithium) et le LiPDI (1-pentafluoroéthyl-4,5-dicarbonitrile-imidazolate de lithium), ainsi que cela est enseigné dans le document WO 2010/023413. Ces sels présentent l'avantage de posséder moins d'atomes de fluor et de comporter des liaisons fortes carbone-fluor en lieu et place des liaisons plus faibles phosphore-fluor du LiPF6. En outre, ces sels présentent de très bonnes conductivités de l'ordre de 6 mS/cm, une très bonne dissociation entre l'anion imidazolate et le cation lithium.

Bukowska et al. (Polish Journal of Chemistry, vol 78, n°3, 2004, p. 417-422) décrit une méthode de préparation du 2-trifluoromethyl-4,5-dicyanoimidazole en deux étapes avec purification intermédiaire.

Niedzicki et al. (Electrochimica Acta, vol. 55, n° 4, 2010, p. 1450-1454) décrit la préparation de sel d'imidazolate en une seule étape.

Le document WO 2010/023413 propose plusieurs voies de synthèse pour la fabrication de ces anions pentacycliques, dont l'une consiste en la condensation du diaminomaléonitrile (DAMN) sur un dérivé d'acide tel qu'un anhydride d'acide fluoré, suivie d'un échange proton/lithium. La condensation est réalisée en une seule étape.

Le rendement maximal de sel de lithium obtenu avec les voies de synthèse connues est d'environ 70 %. Les impuretés présentes nécessitent de lourdes étapes de purification en aval, ce qui représente un frein à une possible industrialisation de ce type de sel de lithium pour une utilisation en tant que sel d'électrolyte de batteries Li-ion.

Par conséquent, il existe un réel besoin de mettre au point un procédé permettant d'obtenir des sels de lithium tels que le LiTDI ou le LiPDI avec un meilleur rendement.

### RESUME DE L'INVENTION

L'invention concerne en premier lieu un procédé de préparation d'un composé imidazole de formule : 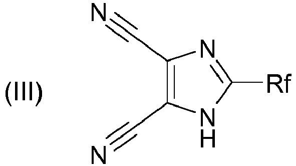 dans laquelle Rf est un groupement alkyle ou alkoxyle fluoré comprenant de 1 à 5 atomes de carbone, le procédé comprenant :
(a) la réaction du diaminomaléonitrile de formule : 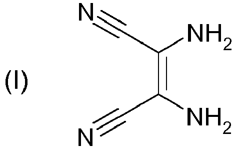 avec le composé de formule : 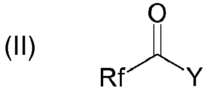 dans laquelle Y représente un atome de chlore ou le groupement OCORf, pour former le composé amide salifié de formule (IVa) et/ou l'amine correspondante (IVb), à une température T₁. 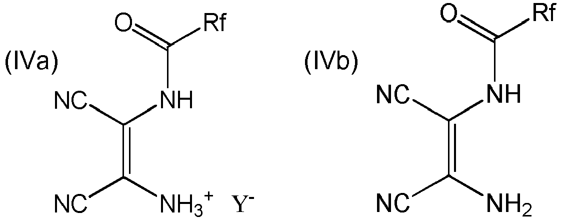
(b) la déshydratation du composé amide salifié de formule (IVa) et/ou l'amine correspondante de formule (IVb) pour former le composé imidazole de formule (III), à une température T₂ supérieure à T₁.

Selon un mode de réalisation, Rf représente CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F₇, C₄H₄F₅, C₅F₁₁, C₃F₆OCF₃, C₂F₄OCF₃, C₂H₂F₂OCF₃ ou CF₂OCF₃ de préférence CF₃, C₂F₅, C₂F₄OCF₃, C₂H₂F₂OCF₃ ou CF₂OCF₃.

Selon un mode de réalisation, T₁ vaut de 0 à 80°C, de préférence de 10 à 50°C, plus préférentiellement de 20 à 30°C.

Selon un mode de réalisation, T₂ vaut de 30 à 180°C, de préférence de 60 à 150°C, plus préférentiellement de 75 à 140°C.

Selon un mode de réalisation, l'étape (a) dure de 1 à 12 heures, de préférence de 1 à 3 heures, et / ou l'étape (b) dure de 1 à 12 heures, de préférence de 1 à 3 heures.

Selon un mode de réalisation, le diaminomaléonitrile et le composé de formule (II) sont dissous dans un solvant préalablement à l'étape (a), le solvant étant de préférence le 1,4-dioxane.

Selon un mode de réalisation, la température T₂ correspond à la température d'ébullition du solvant.

L'invention concerne également un procédé de préparation d'un composé imidazolate de lithium de formule : 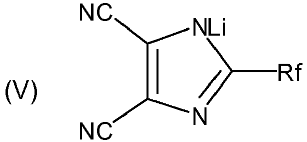 dans laquelle Rf est un groupement alkyle ou alkoxyle fluoré comprenant de 1 à 5 atomes de carbone, le procédé comprenant :
(a) la préparation du composé imidazole de formule : 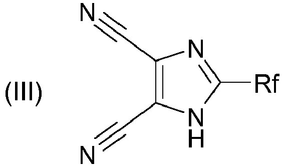 selon le procédé décrit ci-dessus ; et
(b) la réaction du composé imidazole de formule (III) avec une base lithiée.

Selon un mode de réalisation, la base lithiée est choisie parmi l'hydrure de lithium, le carbonate de lithium, l'hydroxyde de lithium et les combinaisons de ceux-ci.

L'invention concerne également un procédé de fabrication d'une composition électrolyte, comprenant la préparation d'imidazolate de lithium de formule (V) selon le procédé décrit ci-dessus, et la dissolution de celui-ci dans un solvant.

L'invention concerne également un procédé de fabrication d'une batterie ou d'une cellule de batterie, comprenant la fabrication d'une composition électrolyte selon le procédé décrit ci-dessus et l'insertion de cette composition électrolyte entre une anode et une cathode.

La présente invention permet de surmonter les inconvénients de l'état de la technique. Elle fournit plus particulièrement un procédé permettant d'obtenir des sels de lithium tels que le LiTDI ou le LiPDI avec un meilleur rendement.

Ceci est accompli grâce à la mise au point d'un procédé de préparation de 4,5-dicarbonitrile-imidazole fluoré par réaction du DAMN avec un dérivé d'acide fluoré en deux étapes qui sont effectuées à des températures différentes, la température de la deuxième étape étant supérieure à la température de la première étape.

Ainsi, on produit de manière stable un composé amide salifié et/ou amine correspondante qui est un intermédiaire de réaction (le composé de formule (IVa) et (IVb)), à la première étape, ce composé amide salifié et/ou amine correspondante étant ensuite déshydraté pour former l'imidazole, lors de la deuxième étape.

Sans vouloir être lié par une théorie, on estime que le faible rendement de production d'imidazole observé dans l'état de la technique est dû à la polymérisation du DAMN par chauffage, particulièrement en milieu acide.

Or, des analyses thermiques ont permis de mettre en évidence que le composé amide salifié et/ou l'amine correspondante intermédiaire est plus stable thermiquement que le DAMN. Le DAMN subit une forte dégradation à partir de 188°C, alors que l'amide salifié intermédiaire et/ou l'amine correspondante subit ou subissent d'abord une déshydratation, puis une dégradation qu'à partir de 210°C.

Etant donné d'une part la plus grande stabilité thermique du composé amide salifié intermédiaire et/ou l'amine correspondante par rapport au DAMN, et d'autre part le fait que la fonction C=O du composé amide a tendance à désactiver la double-liaison C=C et que dans le cas de l'amide salifié l'amine salifiée est un moins bon nucléophile; cela permet ainsi de défavoriser la polymérisation. Le procédé selon l'invention permet : dans un premier temps, de former le composé amide salifié intermédiaire et/ou l'amine correspondante de manière stable, à une température relativement faible à laquelle la polymérisation du DAMN est essentiellement évitée ; et dans un deuxième temps, de déshydrater le composé amide salifié et/ou l'amine correspondante à une température plus élevée, en évitant encore la polymérisation du DAMN (celui-ci ayant été déjà consommé) tout comme la polymérisation du composé amide (pour les raisons exposées ci-dessus).

### DESCRIPTION DE MODES DE REALISATION DE L'INVENTION

L'invention est maintenant décrite plus en détail et de façon non limitative dans la description qui suit.

### Préparation du composé imidazole

L'invention prévoit la préparation du composé imidazole de formule (III) à partir de DAMN de formule (I) et d'un dérivé d'acide fluoré de formule (II), selon le schéma général suivant :

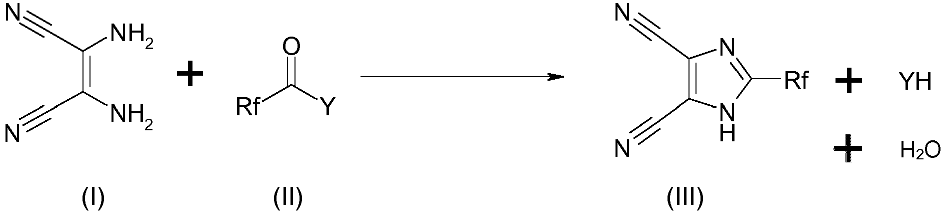

Dans ce schéma, Rf est un groupement alkyle ou alkoxyle fluoré (c'est à dire un groupement alkyle ou alkoxyle comprenant un ou plusieurs substituants fluor), comprenant de 1 à 5 atomes de carbone tels que CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F₇, C₄H₄F₅, C₅F₁₁, C₃F₆OCF₃, C₂F₄OCF₃, C₂H₂F₂OCF₃ ou CF₂OCF₃ de préférence CF₃, C₂F₅, C₂F₄OCF₃, C₂H₂F₂OCF₃ ou CF₂OCF₃.

Par ailleurs, Y représente un atome de chlore (auquel cas le composé de formule (II) est un chlorure d'acyle) ou le groupement OCORf (auquel cas le composé de formule (II) est un anhydride.

Cette réaction est effectuée en deux étapes.

La première étape est effectuée à une température T₁ qui est de 0 à 80°C, de préférence de 10 à 50°C, plus préférentiellement de 20 à 30°C, par exemple environ 25°C. Cette première étape permet de produire le composé amide salifié de formule (Iva) et/ou l'amine correspondante de formule (IVb) :

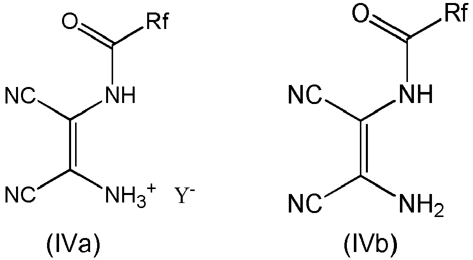

La durée de cette première étape est de préférence de 1 à 12 heures, plus particulièrement de 1 à 3 heures, par exemple d'environ 2 heures.

La réaction est de préférence effectuée en dissolvant les réactifs dans un solvant, par exemple le dioxane, le toluène ou le diméthylformamide et notamment le 1,4-dioxane. Avantageusement, les deux étapes sont mises en oeuvre dans le même solvant.

La concentration du DAMN dans le milieu réactionnel est de préférence de 0,001 à 2 mol/L, plus préférentiellement de 0,1 mol/L à 1 mol/L. Le rapport molaire du composé (I) sur le composé (II) est de préférence de 0,25 à 1,5, plus préférentiellement de 0,5 à 1,25.

La deuxième étape est effectuée à une température T₂ qui est supérieure à T₁. De préférence, T₂ est supérieure à T₁ d'au moins 10°C, ou d'au moins 20°C, ou d'au moins 30°C, ou d'au moins 40°C, ou d'au moins 50°C, ou d'au moins 60°C, ou d'au moins 70°C.

Selon un mode de réalisation particulier, la température T₂ correspond à la température d'ébullition du solvant utilisé.

De préférence, T₂ est de 30 à 180°C, plus particulièrement de 60 à 150°C, plus préférentiellement de 75 à 140°C, par exemple d'environ 100 ou 101°C (ce qui correspond à la température d'ébullition du 1,4-dioxane).

La concentration du composé (IVa) et/ou (IVb) dans le milieu réactionnel lors de la deuxième étape est de préférence de 0,001 à 2 mol/L, plus préférentiellement de 0,05 mol/L à 0,75 mol/L.

La deuxième étape est effectuée immédiatement à la suite de la première étape sans purification intermédiaire et avantageusement sans aucune étape de séparation, simplement en modifiant la température du mélange réactionnel, par chauffage.

Dans le cas où Y = Cl, l'amide est salifié par ajout d'un acide carboxylique qui permet également d'améliorer le rendement de la deuxième étape par catalyse acide. Les acides utilisés sont par exemple de l'acide trifluoroacétique, de l'acide acétique ou de l'acide benzoïque et de préférence de l'acide trifluoroacétique.

Le rapport molaire du composé (IVa) et/ou (IVb) sur le catalyseur est de préférence de 0,5 à 20, plus préférentiellement de 1 à 10.

La température de la réaction T₁ peut être constante tout au long de la première étape, et la température de la réaction T₂ peut être constante tout au long de la deuxième étape, mais ce n'est pas nécessairement le cas. On peut par exemple prévoir une température croissante tout au long de la réaction, ou tout au long de la première étape seulement. Dans de tels cas, la condition selon laquelle T₂ est supérieure à T₁ signifie que la température sur l'ensemble de la deuxième étape est supérieure à la température sur l'ensemble de la première étape, c'est-à-dire encore que la température minimale atteinte lors de la deuxième étape est supérieure à la température maximale atteinte lors de la première étape.

Une période de transition est nécessaire pour passer de la première étape à la deuxième étape et pour opérer le changement de température requis. Cette période de transition présente de préférence une durée inférieure à 1 heure, par exemple inférieure à 30 minutes, par exemple inférieure à 20 minutes, par exemple inférieure à 10 minutes, par exemple inférieure à 5 minutes.

A l'issue de cette réaction, le composé imidazole de formule (III) est de préférence isolé et purifié, par exemple en évaporant le solvant, en ajoutant de l'eau, en extrayant la phase aqueuse obtenue (par exemple avec de l'acétate d'éthyle) et en récupérant les phases organiques.

### Préparation de l'imidazolate de lithium

L'imidazolate de lithium de formule : 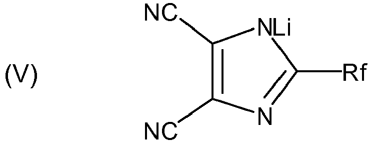 est préparé à partir du composé imidazole de formule (III), en faisant réagir celui-ci avec une base lithiée, de préférence choisie parmi l'hydrure de lithium, le carbonate de lithium, l'hydroxyde de lithium et les combinaisons de ceux-ci.

Par exemple, lorsque le composé imidazole a été isolé et purifié comme décrit ci-dessus à l'issue de la réaction, il est possible d'extraire les phases organiques obtenues avec une solution aqueuse de la base lithiée. La phase aqueuse peut ensuite être évaporée (après un traitement optionnel au charbon actif).

La phase organique contient donc le composé (III) ainsi que le résidu YH et le catalyseur acide en solution dans le solvant de réaction. Le composé (III) est alors à une concentration qui est de préférence de 0,01 à 5 mol/L, plus préférentiellement de 0,1 à 3 mol/L. La concentration en base lithiée dans la phase aqueuse est de préférence de 0,01 à 10 mol/L, plus préférentiellement de 0,1 à 5 mol/L.

Le sel de lithium obtenu est par exemple le LiTDI lorsque Rf représente un groupement trifluorométhyle, et le LiPDI lorsque Rf représente un groupement pentafluoroéthyle.

### Préparation d'un électrolyte

Les composés de formule (V) préparés comme décrit ci-dessus, et notamment le LiTDI et le LiPDI, peuvent être utilisés pour la préparation d'un électrolyte, en les dissolvant dans un solvant approprié.

Les composés de formule (V) sont par exemple dissous dans un mélange composés de 1 à 5 constituants choisis parmi les carbonates suivants : l'éthylene carbonate, le dimethylcarbonate, l'éthylméthylcarbonate, le diéthylcarbonate, le propylene carbonate ; et parmi les glymes suivants : l'éthylène glycol diméthyléther, le diéthylène glycol diméthyléther, le dipropylène glycol diméthyléther, le diéthylène glycol diéthyléther, le triéthylène glycol diméthyléther, le diéthylène glycol dibutyléther, le tétraéthylène glycol diméthyléther et le diéthylène glycol t-buthylméthyléther. Les proportions en masse de chacun des constituants sont de préférence comprises entre 1 et 10 par rapport au constituant en plus faible quantité, plus préférentiellement entre 1 et 8.

La concentration en composé de formule (V) dans l'électrolyte est de préférence de 0,1 mol/L à 5 mol/L, plus préférentiellement de 0,2 mol/L à 2,5 mol/L.

Cet électrolyte peut ensuite être utilisé pour la fabrication de batteries ou de cellules de batterie, en le disposant entre une cathode et une anode, de manière connue en soi.

### EXEMPLE

L'exemple suivant illustre l'invention sans la limiter.

### Synthèse de LiTDI

Dans un ballon de 200 mL, 1,25 g de diaminomaléonitrile sont dissous dans 45 mL de 1,4-dioxane. De l'anhydride d'acide trifluoroacétique (1,6 mL) est ensuite ajouté à cette solution. Le milieu réactionnel est laissé sous agitation à 25°C pendant deux heures ce qui correspond à la première étape du schéma réactionnel ci-dessus. Le milieu réactionnel est ensuite chauffé au reflux du dioxane pendant deux heures pour permettre la déshydratation du composé amide formé lors de la première étape, qui est catalysée par l'acide trifluoroacétique résiduel obtenu lors de la première étape.

Le milieu réactionnel est alors évaporé. De l'eau (60 mL) est ensuite ajoutée et la phase aqueuse obtenue est extraite avec 2 x 50 mL d'acétate d'éthyle. Les phases organiques sont alors rassemblées et extraites avec une solution aqueuse de carbonate de lithium (0,5 g de Li₂CO₃ dans 60 mL d'eau).

La phase aqueuse obtenue étant colorée, elle est décolorée à l'aide d'un traitement au charbon actif. Après traitement, cette phase aqueuse est évaporée et donne 2,01 g de sel de lithium ce qui correspond à un rendement de 90,5 %.

## Revendications

1. Procédé de préparation d'un composé imidazole de formule : 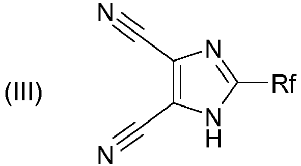 dans laquelle Rf est un groupement alkyle ou alkoxyle fluoré comprenant de 1 à 5 atomes de carbone,
le procédé comprenant :
(a) la réaction du diaminomaléonitrile de formule : 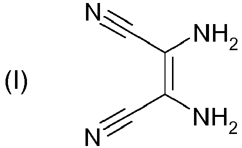 avec le composé de formule : 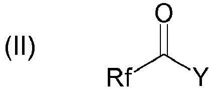 dans laquelle Y représente un atome de chlore ou le groupement OCORf, en présence d'un solvant, pour former le composé amide salifié de formule (IVa) et /ou l'amine correspondante de formule (IVb) : 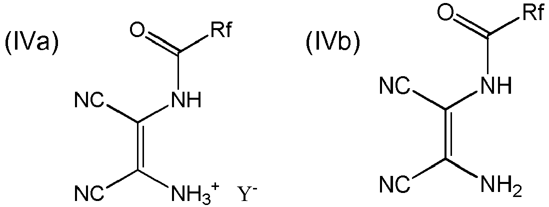 à une température T₁, et
(b) la déshydratation du composé amide salifié de formule (IVa) et/ou l'amine correspondante (IVb) pour former le composé imidazole de formule (III), à une température T₂ supérieure à T_{1,} dans lequel la deuxième étape est effectuée immédiatement à la suite de la première étape sans purification intermédiaire.

2. Procédé selon la revendication 1, dans lequel Rf représente CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F₇, C₄H₄F₅, ou C₅F₁₁, de préférence CF₃, ou C₂F₅,.

3. Procédé selon la revendication 1 ou 2, dans lequel T₁ vaut de 0 à 80°C, de préférence de 10 à 50°C, plus préférentiellement de 20 à 30°C.

4. Procédé selon l'une des revendications 1 à 3, dans lequel T₂ est de 30 à 180°C, de préférence de 60 à 150°C, plus préférentiellement de 75 à 140°C.

5. Procédé selon l'une des revendications 1 à 4, dans lequel l'étape (a) dure de 1 à 12 heures, de préférence de 1 à 3 heures, et / ou l'étape (b) dure de 1 à 12 heures, de préférence de 1 à 3 heures.

6. Procédé selon l'une des revendications 1 à 5, dans lequel les étapes (a) et (b) sont mises en oeuvre dans le même solvant.

7. Procédé selon l'une des revendications 1 à 6, dans lequel le diaminomaléonitrile et le composé de formule (II) sont dissous dans un solvant préalablement à l'étape (a), le solvant étant de préférence le 1,4-dioxane.

8. Procédé selon l'une des revendications 1 à 7, dans lequel la température T₂ correspond à la température d'ébullition du solvant.

9. Procédé selon l'une quelconque des revendications 1 à 8 **caractérisé en ce que** le produit formé à l'étape (a) est le composé de formule (IVa).

10. Procédé selon l'une quelconque des revendications 1 à 9 **caractérisé en ce que** le produit formé à l'étape (a) est le composé de formule (IVb).

11. Procédé de préparation d'un composé imidazolate de lithium de formule : 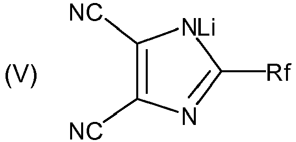 dans laquelle Rf est un groupement alkyle fluoré comprenant de 1 à 5 atomes de carbone, le procédé comprenant :
(a) la préparation du composé imidazole de formule : 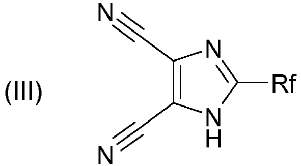 selon le procédé de l'une des revendications 1 à 10 ; et
(b) la réaction du composé imidazole de formule (III) avec une base lithiée.

12. Procédé selon la revendication 11, dans lequel la base lithiée est choisie parmi l'hydrure de lithium, le carbonate de lithium, l'hydroxyde de lithium et les combinaisons de ceux-ci.

13. Procédé de fabrication d'une composition électrolyte, comprenant la préparation d'imidazolate de lithium de formule (V) selon le procédé de la revendication 11 ou 12, et la dissolution de celui-ci dans un solvant.

14. Procédé de fabrication d'une batterie ou d'une cellule de batterie, comprenant la fabrication d'une composition électrolyte selon le procédé de la revendication 13 et l'insertion de cette composition électrolyte entre une anode et une cathode.

## Patentansprüche

1. Verfahren zur Herstellung einer Imidazolverbindung der Formel: 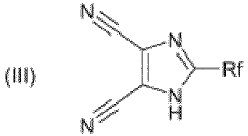 wobei Rf eine fluorierte Alkyl- oder Alkoxylgruppe ist, die 1 bis 5 Kohlenstoffatome umfasst,
wobei das Verfahren Folgendes umfasst:
(a) die Reaktion des Diaminomaleonitrils der Formel: 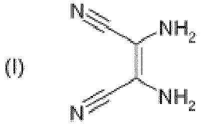 mit der Verbindung der Formel: 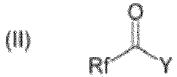 wobei Y ein Chloratom oder die OCORf-Gruppe darstellt, in Gegenwart eines Lösungsmittels, wodurch die als Salz vorliegende Amidverbindung der Formel (IVa) und/oder das entsprechende Amin der Formel (IVb): 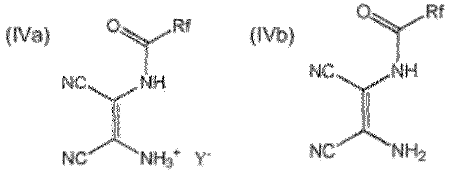 bei einer Temperatur T₁ gebildet wird, und
(b) die Dehydratation der als Salz vorliegenden Amidverbindung der Formel (IVa) und/oder des entsprechenden Amins (IVb), wodurch die Imidazolverbindung der Formel (III) bei einer Temperatur T₂ gebildet wird, die höher als T₁ ist, wobei der zweite Schritt unmittelbar nach dem ersten Schritt ohne Zwischenreinigung durchgeführt wird.

2. Verfahren nach Anspruch 1, wobei Rf CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F₇, C₄H₄F₅ oder C₅F₁₁, vorzugsweise CF₃ oder C₂F₅ darstellt.

3. Verfahren nach Anspruch 1 oder 2, wobei T₁ 0 bis 80 °C, vorzugsweise 10 bis 50 °C, noch mehr bevorzugt 20 bis 30 °C beträgt.

4. Verfahren nach einem der Ansprüche 1 bis 3, wobei T₂ 30 bis 180 °C, vorzugsweise 60 bis 150 °C, noch mehr bevorzugt 75 bis 140 °C beträgt.

5. Verfahren nach einem der Ansprüche 1 bis 4, wobei Schritt (a) 1 bis 12 Stunden, vorzugsweise 1 bis 3 Stunden dauert und/oder Schritt (b) 1 bis 12 Stunden, vorzugsweise 1 bis 3 Stunden dauert.

6. Verfahren nach einem der Ansprüche 1 bis 5, wobei die Schritte (a) und (b) im selben Lösungsmittel erfolgen.

7. Verfahren nach einem der Ansprüche 1 bis 6, wobei das Diaminomaleonitril und die Verbindung der Formel (II) vor Schritt (a) in einem Lösungsmittel gelöst werden, wobei es sich beim Lösungsmittel vorzugsweise um 1,4-Dioxan handelt.

8. Verfahren nach einem der Ansprüche 1 bis 7, wobei die Temperatur T₂ der Siedetemperatur des Lösungsmittels entspricht.

9. Verfahren nach einem der Ansprüche 1 bis 8, **dadurch gekennzeichnet, dass** es sich bei dem in Schritt (a) gebildeten Produkt um die Verbindung der Formel (IVa) handelt.

10. Verfahren nach einem der Ansprüche 1 bis 9, **dadurch gekennzeichnet, dass** es sich bei dem in Schritt (a) gebildeten Produkt um die Verbindung der Formel (IVb) handelt.

11. Verfahren zur Herstellung einer Lithiumimidazolatverbindung der Formel: 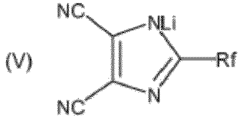 wobei Rf eine fluorierte Alkylgruppe ist, die 1 bis 5 Kohlenstoffatome umfasst, wobei das Verfahren Folgendes umfasst:
(a) die Herstellung der Imidazolverbindung der Formel: 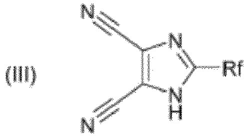 gemäß dem Verfahren eines der Ansprüche 1 bis 10 und
(b) die Reaktion der Imidazolverbindung der Formel (III) mit einer Lithiumbase.

12. Verfahren nach Anspruch 11, wobei die Lithiumbase aus Lithiumhydrid, Lithiumcarbonat, Lithiumhydroxid und Kombinationen davon ausgewählt ist.

13. Verfahren zur Herstellung einer Elektrolytzusammensetzung, umfassend die Herstellung des Lithiumimidazolats der Formel (V) gemäß dem Verfahren von Anspruch 11 oder 12 und dessen Auflösung in einem Lösungsmittel.

14. Verfahren zur Herstellung einer Batterie oder einer Batteriezelle, umfassend die Herstellung einer Elektrolytzusammensetzung gemäß dem Verfahren von Anspruch 13 und das Einführen dieser Elektrolytzusammensetzung zwischen einer Anode und einer Kathode.

## Claims

1. A process for preparing an imidazole compound of formula: 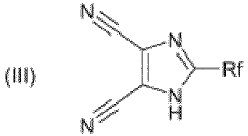 in which Rf is a fluoro alkyl or alkoxyl group comprising from 1 to 5 carbon atoms,
the process comprising:
(a) the reaction of diaminomaleonitrile of formula: 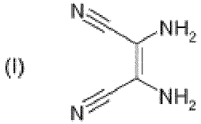 with the compound of formula: 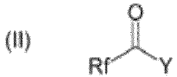 in which Y represents a chlorine atom or the group OCORf, in the presence of a solvent, to form the salified amide compound of formula (IVa) and/or the corresponding amine of formula (IVb): 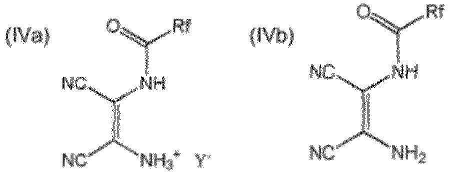 at a temperature T₁, and
(b) dehydration of the salified amide compound of formula (IVa) and/or the corresponding amine (IVb) to form the imidazole compound of formula (III), at a temperature T₂ above T₁, wherein the second step is performed immediately following the first step without intermediate purification.

2. The method as claimed in claim 1, wherein Rf represents CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F₇, C₄H₄F₅, or C₅F₁₁, preferably CF₃, or C₂F₅.

3. The process as claimed in claim 1 or 2, wherein T₁ is from 0 to 80°C, preferably from 10 to 50°C, more preferentially from 20 to 30°C.

4. The process as claimed in one of claims 1 to 3, wherein T₂ is from 30 to 180°C, preferably from 60 to 150°C, more preferentially from 75 to 140°C.

5. The process as claimed in one of claims 1 to 4, wherein step (a) lasts from 1 to 12 hours, preferably from 1 to 3 hours, and/or step (b) lasts from 1 to 12 hours, preferably from 1 to 3 hours.

6. The process as claimed in one of claims 1 to 5, wherein steps (a) and (b) are performed in the same solvent.

7. The process as claimed in one of claims 1 to 6, wherein diaminomaleonitrile and the compound of formula (II) are dissolved in a solvent prior to step (a), the solvent preferably being 1,4-dioxane.

8. The process as claimed in one of claims 1 to 7, wherein the temperature T₂ corresponds to the boiling point of the solvent.

9. The process as claimed in any one of claims 1 to 8, **characterised in that** the product formed in step (a) is the compound of formula (IVa).

10. The process as claimed in any one of claims 1 to 9, **characterised in that** the product formed in step (a) is the compound of formula (IVb).

11. A process for preparing a lithium imidazolate compound of formula: 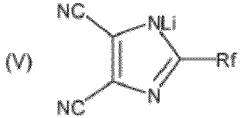 wherein Rf is a fluoro alkyl group comprising from 1 to 5 carbon atoms, the process comprising:
(a) preparation of the imidazole compound of formula: 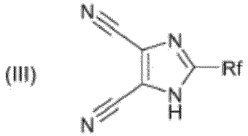 according to the process of one of claims 1 to 10; and
(b) reaction of the imidazole compound of formula (III) with a lithium base.

12. The process as claimed in claim 11, wherein the lithium base is chosen from lithium hydride, lithium carbonate and lithium hydroxide, and combinations thereof.

13. A process for manufacturing an electrolyte composition, comprising the preparation of the lithium imidazolate of formula (V) according to the process of claim 11 or 12, and dissolution of this compound in a solvent.

14. A process for manufacturing a battery or a battery cell, comprising the manufacture of an electrolyte composition according to the process of claim 13 and the insertion of this electrolyte composition between an anode and a cathode.
